# EUROPÄISCHE PATENTSCHRIFT

(11) **EP 1 216 997 B1**
(45) Veröffentlichungstag und Bekanntmachung des Hinweises auf die Patenterteilung: **15.02.2006**
(21) Anmeldenummer: 01129212.5
(22) Anmeldetag: 10.12.2001
(51) Int. Cl.: C07D 277/32, C07D 277/68, C07D 277/60, C07K 1/10

(54) **2-Brom/Chlor-thiazoliumsalze und ihre Verwendung als Kondensationsreagenzien**
2-bromo/chloro-thiazolium salts and their use as condensation agents
Sels de 2-bromo/chloro-thiazolium et leur utilisation comme agents de condensation

(30) Priorität: 20.12.2000 DE 10063495; 02.07.2001 DE 10131572
(43) Veröffentlichungstag der Anmeldung: 26.06.2002
(73) Patentinhaber: LANXESS Deutschland GmbH, 51369 Leverkusen (DE)
(72) Erfinder: Rudolph, Joachim, Dr., Guilford CT 06437 (US); Wischnat, Ralf, Dr., 51061 Köln (DE)

(56) Entgegenhaltungen:
- WO-A-97/48687
- CHEMICAL ABSTRACTS, vol. 133, no. 20, 12. November 2000 (2000-11-12) Columbus, Ohio, US; abstract no. 282083t, Seite 800; XP002191977 -& DATABASE CAPLUS [Online] CHEMICAL ABSTRACTS SERVICE, COLUMBUS, OHIO, US; Database accession no. 2000:749442 XP002191978 & CN 1 243 828 A (FAMING ZHUANLI SHENQING GONGKAI SHUOMINGSHU) 9. Februar 2000 (2000-02-09)
- LI P ET AL: "A novel thiazolium type peptide coupling reagent for hindered amino acids" TETRAHEDRON LETTERS, Bd. 40, Nr. 47, 19. November 1999 (1999-11-19), Seiten 8301-8304, XP004188432
- LI P ET AL: "Total synthesis of cyclosporin O both in solution and in the solid phase using novel thiazolium-, immonium-, and pyridinium-type coupling reagents: BEMT, BDMP, and BEP" JOURNAL OF ORGANIC CHEMISTRY, Bd. 65, Nr. 10, 19. Mai 2000 (2000-05-19), Seiten 2951-2958, XP002191973
- SUGIMOTO H ET AL: "Activation of dithiocarbamate by 2-halothiazolium salts" JOURNAL OF ORGANIC CHEMISTRY, Bd. 53, Nr. 10, 13. Mai 1988 (1988-05-13), Seiten 2263-2267, XP002191974
- BALLI H ET AL: "Azidiniumsalze, I. Synthese quasiaromatischer Azidocyclimonium-fluoroborate" JUSTUS LIEBIGS ANNALEN DER CHEMIE, Bd. 647, 31. Oktober 1961 (1961-10-31), Seiten 1-10, XP000881319
- FRASER P J ET AL: "Carbene complexes of iridium, rhodium, manganese, chromium, and ironcntaining thiazolidinylidene and pyridinylidene ligands" JOURNAL OF THE CHEMICAL SOCIETY, DALTON TRANSACTIONS, 1974, Seiten 760-764, XP002191975
- DESTEVENS G ET AL: "Investigations in heterocycles. I. Cycloalkeno[d]thiazolin-2-ones and their analgetic properties" JOURNAL OF THE AMERICAN CHEMICAL SOCIETY, Bd. 79, Nr. 19, 5. Oktober 1957 (1957-10-05), Seiten 5263-5270, XP002191976
- CHEMICAL ABSTRACTS, vol. 57, no. 8, 15. Oktober 1962 (1962-10-15) Columbus, Ohio, US; abstract no. 9836d, BHALLA J S ET AL: "Syntheses of some 2-bromothiazoles" XP002201065 & J. SCI. IND. RES. (INDIA), Bd. 21B, 1962, Seiten 291-292,
- CHEMICAL ABSTRACTS, vol. 55, no. 13, 26. Juni 1961 (1961-06-26) Columbus, Ohio, US; abstract no. 12388f, RALHAN N K ET AL: "Thiazoles. IV. Preparation of some 2-bromothiazoles" XP002201066 & J. INDIAN CHEM. SOC., Bd. 37, 1960, Seiten 773-774,
- CHEMICAL ABSTRACTS, vol. 50, no. 14, 25. Juli 1956 (1956-07-25) Columbus, Ohio, US; abstract no. 10711h, BARIANA D S ET AL: "Sudies on thiazoles" XP002201067 & J. INDIAN CHEM. SOC., Bd. 32, 1955, Seiten 427-430,
- CHEMICAL ABSTRACTS, vol. 52, no. 7, 10. April 1958 (1958-04-10) Columbus, Ohio, US; abstract no. 5383d, SHARMA G M ET AL: "Thiazoles. II. Synthesis of some 2-chloro- and 2-hydroxythiazoles" XP002201068 & J. SCI. IND. RESEARCH (INDIA), Bd. 16B, 1957, Seiten 411-413,
- GREGORY J T ET AL: "Reactions of 3-thiocyano-2-butanone. I. The preparation of 2-substituted-4,5-dimethylthiazoles" JOURNAL OF THE AMERICAN CHEMICAL SOCIETY, Bd. 74, Nr. 7, 5. April 1952 (1952-04-05), Seiten 1719-1720, XP002201062
- DHAMI K S ET AL: "Studies on thiazoles. V. Synthesis of some 2-chloro- and 2-hydroxythiazoles" JOURNAL OF ORGANIC CHEMISTRY, Bd. 27, Nr. 6, Juni 1962 (1962-06), Seiten 2147-2149, XP002201063
- MERIJANIAN A ET AL: "Steric effects of ortho substituents on acid-catalyzed cyclization of thiocyanatoacetophenones" JOURNAL OF ORGANIC CHEMISTRY, Bd. 51, Nr. 4, 21. Februar 1986 (1986-02-21), Seiten 543-545, XP002201064
- DATABASE CROSSFIRE BEILSTEIN [Online] Beilstein Institut zur Förderung der Chemischen Wissenschaften, Frankfurt am Main, DE; Database accession no. Reaction ID 1255281 XP002201069 & INDIAN J. CHEM., Bd. 10, 1972, Seiten 605-607,

## Beschreibung

Die vorliegende Erfindung betrifft neue Bromthiazoliumsalze und deren Verwendung als Kondensationsreagenzien, insbesondere als Peptidkupplungsreagenzien, Verfahren zu deren Herstellung sowie in diesem Verfahren benötigte Zwischenprodukte.

Verschiedene Thiazoliumsalze sind bereits aus der Literatur bekannt (vgl. z.B. Chemical Abstracts, Vol. 133, No. 20, 12, 12. November 2000, 282 083 t; Li et al., J. Org. Chem. 2000, 65, 2951-2958; Sugimoto et al., J. Org. Chem. 1988, 53, 2263-2267; Balli et al., Justus Liebeigs Annalen der Chemie 1961, Bd. 647, 1-10; DeStevens et al, J.Am.Chem.Soc. 1957, Vol. 79, No. 19, 5263-5270).

Für die Herstellung leistungsfähiger Peptidkupplungsreagenzien wie z. B. des erstmals 1999 von P. Li publizierten Peptidkupplungsreagenzes 2-Brom-3-ethyl-4-methyl-thiazolium-tetrafluoroborats (BEMT, siehe Tetrahedron Lett. 1999, 40, 8301-8304) werden als unmittelbare Vorstufe Bromthiazole wie z. B. das 2-Brom-4-methylthiazol benötigt. Die für die Herstellung dieser Verbindungen, insbesondere des 2-Brom-4-methylthiazols, bekannten Verfahren sind nicht befriedigend und ungeeignet für die Herstellung im Mol- oder größerem Maßstab. Das strukturell ähnliche, aber weniger aktive Reagenz 2-Brom-1-ethyl-pyridinium-tetrafluoroborat (BEP, siehe Chem. Lett. 2000, 204-205) ist zum Beispiel wesentlich leichter herstellbar, länger bekannt und kommerziell erhältlich. Es besteht deshalb ein Bedarf an verbesserten Verfahren zur Herstellung von Peptidkupplungsreagenzien wie BEMT sowie geeigneten Bromthiazolen wie 2-Brom-4-methylthiazol und Wegen zu deren Herstellung.

Ebenso besteht ein Bedarf an weiteren Peptidkupplungsreagenzien, die mit hoher Effizienz eingesetzt werden und mit praktikablen Verfahren hergestellt werden können. So sind z. B. das 2-Chlorthiazol-Derivat des BEMT, 2-Chlor-3-ethyl-4-methyl-thiazol-tetrafluoroborat (CEMT, CAS-Nr. 667-86-7, Dalton Trans. (1974), 7, 760-764) sowie dessen Vorstufe, das 2-Chlor-methylthiazol (CMT, CAS-Nr. 26847-01-8, JP 440 32 406) bekannte Verbindungen. Während jedoch das BEMT bereits als Kupplungsreagenz beschrieben wurde, ist das Chlorderivat (CEMT) bislang nicht als Kupplungsreagenz bekannt geworden. Der Schlüsselbaustein für die Herstellung des CEMT, das CMT, wurde zudem stets über einen Verfahrensweg hergestellt, der wie die Verfahren zur Herstellung des BEMT Nachteile aufweist (Raubenheimer, H. G. et al (1997), Organomet. Chem. 544, 91-100).

2-Brom-4-methylthiazol kann durch Sandmeyer-Reaktion aus 2-Amino-4-methylthiazol hergestellt werden. Die Aufarbeitung ist aufwändig und die Ausbeute mit 32% d. Th. unbefriedigend (vgl. Yakugaku Zasshi 1960, 80, 1795 zitiert in C.A. *55*:10417). Li gibt 1999 (vgl. oben) nur eine Ausbeute für die gesamte Synthesesequenz für die Herstellung von BEMT an, die wenig zufriedenstellend ist. Bei eigenen Versuchen, dies nachzustellen, wurde zusätzlich gefunden, dass ungefähr 30% 2,5-Dibrom-4-methylthiazol als unerwünschtes und schwer abtrennbares Nebenprodukt entsteht. Ein weiterer beschriebener Syntheseweg ist die Bromierung von 4-Methylthiazol. Die Reaktion mit *N*-Bromsuccinimid in Tetrachlormethan hat über die geringe Ausbeute von nur 26% hinaus weitere Nachteile (vgl. Zh. Obshch. Khim. 1957, 27, 726 englische Übersetzung in J. Gen. Chem. USSR S. 799). Zum einen ist das Reagenz bei technischem Einsatz recht teuer, zum anderen die Verwendung von Tetrachlormethan aus Arbeitschutzgründen unerwünscht und in manchen Ländern sogar verboten. Eine Bromierung mit elementarem Brom gelang bisher nicht (vgl. Current Sci. (India), **1952,** *21,* 314 zitiert in C.A. *48*:2046 und Zh. Obshch. Khim.).

Die Synthese von 2-Brom-4-ethylthiazol und 2-Brom-4,5-dimethylthiazol durch Cyclisierung von α-Thiocyanatoalkanonen mit Bromwasserstoffsäure ist aus J. Sci. Ind. Res. Sect. B **1962,** *21,* 291 bekannt. Über eine analoge Synthese von 2-Brom-4-methylthiazol ist bisher jedoch trotz des bestehenden Bedarfs nichts bekannt geworden.

Es wurden nun die neuen Bromthiazoliumsalze der Formel (I) gefunden, 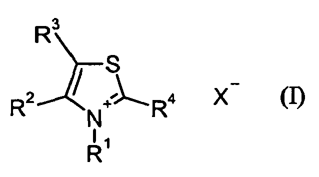
in welcher die Reste R¹, R², R³ und R⁴ sowie das Anion X⁻ nachstehende Bedeutungen besitzen:

| **Verbindung** | **R**^{**1**} | **R**^{**2**} | **R**^{**3**} | **R**^{**4**} | **X**^{**-**} |
|---|---|---|---|---|---|
| K | Methyl | Methyl | H | Br | Br |
| J | Methyl | Methyl | H | Br | CH₃OSO₃⁻ |
| I | Ethyl | -(CH₂)₄- | | Br | BF₄⁻ |

Die Verbindungen der Formel (I) eignen sich in besonderer Weise als Peptidkupplungreagenzien, insbesondere in der Peptidsynthese als Peptidkupplungsreagenzien.

Die neuen Bromthiazoliumsalze der Formel (I) sind besonders zur Verwendung als Kondensationsreagenzien, insbesondere als Peptidkupplungsreagenzien geeignet. Insbesondere eignen sich die Bromthiazoliumsalze der unten genannten Formel (I-2) als Kondensationsreagenzien, insbesondere als Peptidkupplungsreagenzien. Weiterhin eignen sich die neuen Bromthiazoliumsalze der Formel (I), insbesondere diejenigen der Formel (I-2), als Kondensationsreagentien zur Ausbildung einer Amidbindung zwischen einer Carbonsäure oder einem Carbonsäurederivat und einem Amin.

Man erhält die neuen Bromthiazoliumsalze der Formel (I) bzw. der im Folgenden beschriebenen Formeln (I-1) und (I-2) durch die unten beschriebenen Verfahren (A) und (B).
(A) Man erhält die neuen Bromthiazoliumsalze der Formel (I-1) 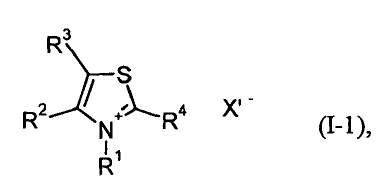
   in welcher
   - R¹, R², R³ und R⁴: eine der vorstehend angegebenen Bedeutungen haben, und
   - X'-: für Chlorid, Bromid oder Methansulfonat steht,

   wenn man
   Bromthiazole der Formel (II) 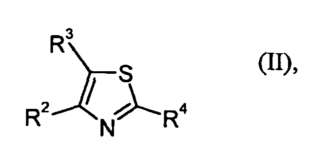
   in welcher
   R², R³ und R⁴ die oben angegebene Bedeutung haben,
   (a) mit Alkylierungsreagenzien der Formel (III)

      R¹-X' (III),

      in welcher
      - R¹: die oben angegebene Bedeutung hat, und
      - X': für Chlor, Brom oder Methylsulfonyloxy steht,

      in Gegenwart eines Verdünnungsmittels umsetzt, oder
   (b) mit Wasserstoffperoxid (H₂O₂), Persäuren oder NaOCl oxidiert.
(B) Man erhält die neuen Bromthiazoliumsalze der Formel (I-2) 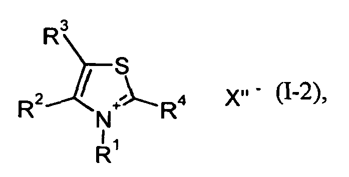
   in welcher
   - R¹, R², R³ und R⁴: die oben angegebene Bedeutung haben, und
   - X"-: für Tetrafluoroborat steht,

   wenn man
   (a) Bromthiazole der Formel (II) 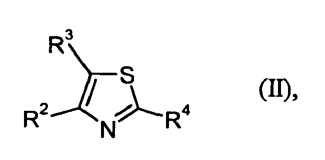
      in welcher
      R², R³ und R⁴ die oben angegebene Bedeutung haben,
      mit Alkylierungsreagenzien der Formel (IV)

      (R¹)₃-O⁺ X"⁻ (IV),

      in welcher
      - R¹ und X"-: die oben angegebene Bedeutung haben,

      in Gegenwart eines Verdünnungsmittels umsetzt, oder wenn man
   (b) Verbindungen der Formel (I-1) 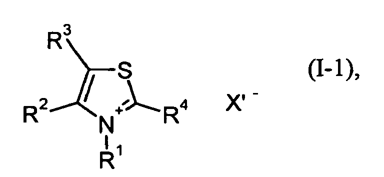
      in welcher
      R¹, R², R³, R⁴ und X'⁻ die oben angegebene Bedeutung haben,

   einsetzt und das Anion X'- mit Tetrafluoroborwasserstoffsäure oder einem mit diesen Säuren beladenen Anionenaustauscher gegen eines mit der o.a. Bedeutung von X"- austauscht.

Die Verbindungen der Formel (II) erhält man, wenn man Verbindungen der Formel (V) 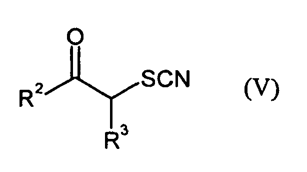
in welcher
R² und R³ eine der oben angegebenen Bedeutungen haben,
mit Bromwasserstoff in Gegenwart eines Verdünnungsmittels umsetzt und anschließend den Bromwasserstoff aus der zunächst erhaltenen Verbindung der Formel (VI) 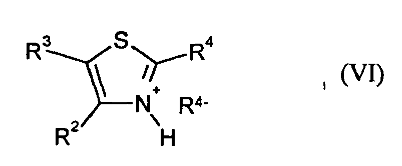
freisetzt.

Das Bromthiazol der Formel 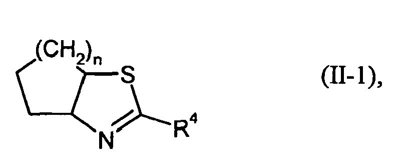
in welchem
- R⁴: für Br und
- n: für 2 steht

ist noch nicht aus der Literatur bekannt und ebenfalls insbesondere Gegenstand dieser Erfindung.

So erhält man das Bromthiazol der Formel (II-1), wenn man wie vorne allgemein beschrieben 2-Thiocyanatocyclohexanon mit Bromwasserstoff in Gegenwart eines Verdünnungsmittels umsetzt und anschließend den Bromwasserstoff aus dem zunächst erhaltenen Hydrobromid freisetzt.

Die Verbindungen der Formel (V) sind bekannt und/oder können nach an sich bekannten Verfahren hergestellt werden (siehe z. B. Schantl et al. 1998, Synth. Commun. 28, 1451-1462, Indian J. Chem., Sect. B (1991), 30, 1152-1155, J. Org. Chem. (1986), 51, 543-545, J. Indian Chem. Soc. (1965), 32, 427, J. Am. Chem. Soc. (1952), 74, 1719, Indian J. Chem. (1967),5, 526).

So erhält man weiter die aus der Literatur bekannte Verbindung 2-Brom-4-methylthiazol gemäß Formel (II), wenn man 1-Thiocyanato-2-propanon mit Bromwasserstoff in Gegenwart eines Verdünnungsmittels umsetzt und anschließend den Bromwasserstoff aus dem zunächst erhaltenen 2-Brom-4-methylthiazoliumbromid freisetzt.

Dieses Verfahren kann als Beispiel für die Herstellung von Verbindungen der Formel (II) wie folgt dargestellt werden:

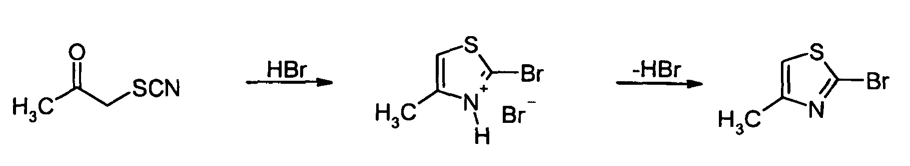

Vorteilhaft ist in diesem Fall, dass diese Synthese in hoher Ausbeute aus einem sehr gut zugänglichen Ausgangsmaterial erfolgt. 1-Thiocyanato-2-propanon ist in zwei Reaktionsschritten durch Chlorierung, Bromierung oder Iodierung von Aceton und anschließende Umsetzung mit Rhodaniden erhältlich (vgl. z.B. Chem. Ber. 1928, 61, 1784).

Die erfindungsgemäße Cyclisierung von Verbindungen der Formel (V) mit Bromwasserstoff wird in Gegenwart eines Verdünnungsmittels durchgeführt. Hierfür kommen bestimmte aprotische organische Lösungsmittel und beliebige Mischungen davon in Betracht. Beispielhaft seien genannt: Alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol. Bevorzugt setzt man Methylenchlorid, Chloroform, 1,2-Dichlorethan, Diethyl- oder tert.-Butylmethylether, besonders bevorzugt Methylenchlorid ein.

Man setzt bei der Cyclisierung pro Mol einer Verbindung der Formel (V) 2,0 bis 10 Mol, bevorzugt 2,1 bis 7 Mol Bromwasserstoff und 0,5 bis 5 1, bevorzugt 1 bis 3 1 Verdünnungsmittel ein.

Die Cyclisierung wird unter Feuchtigkeitsauschluss durchgeführt. Dieses kann man beispielsweise dadurch gewährleisten, dass man handelsübliche trockene Verdünnungsmittel einsetzt oder diese nach den allgemein üblichen Trocknungsmethoden absolutiert, weiterhin trockenen Bromwasserstoff einsetzt und/oder diesen durch eine tiefgekühlte Gasfalle und/oder einen Trockenturm mit einem geeigneten Trockenmittel oder eine Gaswascheinrichtung, wie z.B. eine Waschflasche mit konzentrierter Schwefelsäure, leitet. Die Cyclisierung führt man zweckmäßigerweise so durch, dass man die Verbindung der Formel (V) vorzugsweise in dem Verdünnungsmittel vorlegt und dann den Bromwasserstoff unter Temperaturkontrolle und guter Verteilung einleitet. Die exotherme Reaktion führt man im Allgemeinen bei einer Temperatur von -30 bis +40°C, bevorzugt bei -15 bis +30°C durch. Besonders vorteilhaft ist es, die Temperatur bei der Einleitung von Bromwasserstoff bzw. Chlorwasserstoff zwischen 0 und +10°C zu halten und dann noch ½ bis 15 h bei Raumtemperatur nachreagieren und kristallisieren zu lassen. Die daraus resultierende Verbindung der Formel (VI) kann dann bequem durch ein Fest-Flüssig-Abtrennverfahren, wie beispielsweise Abfiltrieren oder Zentrifugieren, erhalten werden.

Zur Freisetzung des Bromthiazole der Formel (II) aus dem Hydrobromid bzw. dem Hydrochlorid sind schwächere Säureakzeptoren geeignet. Hierzu kommen organische und anorganische Basen in Frage. Hierzu gehören vorzugsweise Alkalimetallcarbonate oder -hydrogencarbonate, wie beispielsweise Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen. Bevorzugt sind Natriumhydrogen- und Kaliumhydrogencarbonat.

Die Freisetzung der Bromthiazole gemäß Formel (II) aus dem Hydrobromid kann ohne vorherigen Trocknungsschritt in dem gleichen Verdünnungsmittel wie die Cyclisierung erfolgen. Hierzu ist es vorteilhaft, beim Abtrennen des Hydrobromids bzw. Hydrochlorids mit etwas Verdünnungsmittel nachzuwaschen, um den überschüssigen Bromwasserstoff weitgehend zu entfernen. Man suspendiert das Bromthiazoliumbromid im Verdünnungsmittel (im Allgemeinen 0,8 bis 31 pro Mol Hydrobromid) und gibt dann die Base hinzu. Bevorzugt setzt man eine wässrige Lösung einer anorganischen Base ein, wie beispielsweise Natriumhydrogencarbonatlösung. Die Konzentration ist dabei unkritisch. Vorzugsweise nimmt man höherkonzentrierte bis gesättigte Lösungen. Pro Mol Hydrobromid setzt man 1,0 bis 1,5 Äquivalente, bevorzugt 1,0 bis 1,2 Äquivalente Base ein. Die Neutralisation führt man im Allgemeinen bei einer Temperatur von -20 bis +30°C, bevorzugt bei -5 bis +10°C durch.

Die Isolierung des Bromthiazols erfolgt nach den üblichen Methoden der organischen Chemie. Vorzugsweise führt man eine Phasentrennung durch und destilliert die organische Phase. Man kann vor der Destillation eine Trocknung mit einem Trockenmittel wie beispielsweise Magnesium- oder Natriumsulfat, Calciumchlorid, Silicagel oder Molekularsieb durchführen.

Die für die erfindungsgemäße Herstellung der 3-Alkyl-2-halogenthiazoliumsalze der Formel (I-1) oder (I-2) durch Alkylierung benötigten Reagenzien der Formeln (III), (VII) und (IV) sind allgemein bekannt bzw. kommerziell erhältlich.

Die erfindungsgemäße Herstellung der 3-Alkyl-2-halogenthiazoliumsalze der Formel (I-1) oder Formel (I-2) wird jeweils in Gegenwart eines Verdünnungsmittels durchgeführt. Hierfür kommen bestimmte aprotische organische Lösungsmittel und beliebige Mischungen davon in Betracht. Beispielhaft genannt seien alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol, Methylcyanid, Aceton, Dimethylformamid, Ethylacetat oder Homologe und Dimethylsulfat, welches gleichzeitig als Alkylierungsmittel verwendet werden kann.

Man setzt im Allgemeinen pro Mol 2-Bromthiazol der Formel (II) 0,8 bis 2, bevorzugt 1,0 bis 1,5 Äquivalente Alkylierungsreagenz der Formel (III) oder (IV) und 0,1 bis 5 1 Verdünnungsmittel ein.

Die Alkylierung führt man unter allgemein üblichen Bedingungen durch. Man arbeitet beispielsweise bei einer Temperatur von -80 bis +100°C. Die bevorzugte Temperatur richtet sich nach der jeweils unterschiedlichen Reaktivität des Reagenzes.

Die Aufarbeitung erfolgt nach allgemein bekannten Methoden der organischen Chemie. Eine bevorzugte Aufarbeitung nach Reaktion in Dichlormethan ist die Kristallisation durch Zugabe von wenig polaren aprotischen Verdünnungsmitteln wie beispielsweise tert.-Butylmethylether, Diethylether oder Hexan. Wenn gewünscht, kann durch Umkristallisation aus Alkoholen wie beispielsweise Methanol, Ethanol oder Isopropanol oder anderen organischen Lösungsmitteln weiter gereinigt werden.

Die Umsetzungen der erfindungsgemäßen Verfahren können bei Normaldruck oder unter erhöhtem Druck durchgeführt werden. Vorzugsweise wird bei Normaldruck gearbeitet. Die Reaktionsdurchführung, Aufarbeitung und Isolierung der Reaktionsprodukte erfolgt, wenn nicht anders angegeben nach allgemein üblichen, bekannten Methoden.

Weitere Details gehen aus den nachfolgenden Beispielen hervor, ohne dass sich dadurch die Erfindung darauf beschränkt.

### Herstellungsbeispiele

### Beispiel 1

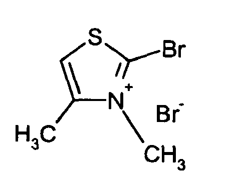

Zu einer Lösung von 1,0g (5,62 mmol) 2-Brom-4-methylthiazol in 4,0 mL N,N-Dimethylformamid werden in einem Druckgefäß bei 25°C 5,33 g (56,2 mmol) Brommethan gegeben. Das Gefäß wird verschlossen und es wird auf 60°C erwärmt. Nach 12 h Rühren wird auf 25°C abgekühlt, und das Lösungsmittel wird im Vakuum entfernt und dreimal mit Ethylacetat zum Entfernen letzter DMF-Spuren codestilliert. Der Rückstand wird aus Isopropanol umkristallisiert.

Ausbeute an 2-Brom-3-methyl-4-methyl-thiazol-bromid (**Verbindung I**): 0,47 g (31 %), weiße Kristalle
Smp.: 260°C (Zersetzung).
¹H-NMR (400 MHz, MeOH-d₃): δ = 2,63 (s, 3H, CH₃ Thiazol), 4,08 (s, 3H, CH₃), 7,98 (s, 1H, Thiazol).
¹³C-NMR (400 MHz, MeOH-d₃): δ = 15,2, 40,4, 122,5, 147,6, 149,3.
MS (ESI⁺): m/z = 192 (⁷⁹Br), 194 (⁸¹Br).

### Beispiel 2

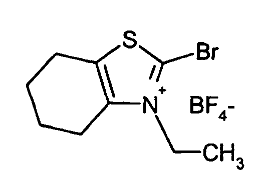

Zu einer Lösung von 11,50 g (0,06 mol) Triethyloxoniumtetrafluoroborat in 50 mL Dichlorethan werden 12, g (0,06 mol) 2-Brom-4,5,6,7-tetrahydro-benzothiazol gelöst in 25 mL Dichlorethan so zugetropft, dass 20°C nicht überschritten werden. Nach beendeter Zugabe wird für 1 h zum Rückfluss erhitzt, anschließend auf Raumtemperatur gekühlt und das Produkt durch Zugabe von 1000 mL Methyl-tert.-butylether zur Kristallisation gebracht und aus Isopropanol umkristallisiert.

Ausbeute an 2-Brom-3-ethyl-4, 5, 6, 7-tetrahydro-benzothiazol-tetrafluoroborat (**Verbindung K**): 16,0 g (86 %), weiße Kristalle.
Smp.: 208°C.
¹H-NMR (400 MHz, CD₃CN): δ = 1,40 (s, 3H, CH₂CH₃), 1,75 - 2,00 (m, 4H, 2 x CH₂), 2,80 (t, 2H, CH₂), 4,40 (q, 2H, CH₂CH₃).
MS (GC/ESI⁺): m/z = 246 (⁷⁹Br), 248 (⁸¹Br)

### Beispiel 3

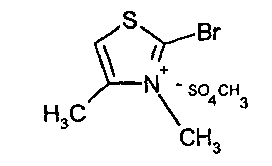

25g (140 mmol) 2-Brom-4-methyl-thiazol werden in 50 ml Dichlorethan gelöst und bei Raumtemperatur mit 8.85 g (70 mmol) Dimethylsulfat versetzt. Anschließend wird zur vollständigen Umsetzung für 1 h auf 50 °C erwärmt. Man kühlt auf Raumtemperatur und versetzt die Lösung mit 300 ml Methyl-tert-butylether. Die Kristalle werden abgesaugt und anschließend aus Isopropanol umkristallisiert.

Es entsteht 2-Brom-3, 4-di-methyl-thiazolium-methylsulfat **(Verbindung J)** in einer Ausbeute von 8.5 g (25.1 % der Theorie).

¹H-NMR (400 MHz, CDCl₃): 2,62 (s, 3H), 3,68 (s, 3H), 4,19 (s, 3H), 8,16 (s, 1H, Thiazol).
EI/MS (m/z): 279 (Br₇₉), 281 (Br₈₁).

### Ausgangsstoffe der Formel (II)

### Beispiel II-A

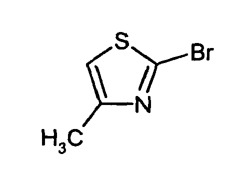

Bei 10°C werden unter Feuchtigkeitsausschluss 11,6 mol Bromwasserstoff in eine Lösung von 280 g (2,30 mol) Thiocyanato-propan-2-on in 4500 ml Dichlormethan so eingeleitet, dass 10°C nicht überschritten werden. Bromwasserstoff wurde dabei nach literaturbekannten Verfahren aus Brom und Tetralin erzeugt. In Abwandlung zur Literaturvorschrift wurde auf die Zugabe von katalytischen Mengen Eisen verzichtet und die Reaktionstemperatur zur Bromwasserstofferzeugung auf 40°C erhöht. Nachdem die Zugabe des Bromwasserstoffs abgeschlossen ist (ca. 6 h), wird 12 h bei Raumtemperatur nachgerührt. Das als Hydrobromid anfallende Produkt wird abgesaugt, mit 1500 ml Dichlormethan gewaschen und erneut in 3000 ml Dichlormethan suspendiert. Nun werden in einem 201-Glasreaktor bei 0°C 2500 ml gesättigte Natriumhydrogencarbonatlösung unter kräftiger Durchmischung (9600 min⁻¹) so zugegeben, dass 5°C nicht überschritten werden. Die organische Phase wird abgetrennt, über MgSO₄ getrocknet und das Lösungsmittel bei 30°C und 50 mbar am Rotationsverdampfer abdestilliert. Der verbleibende Rückstand wird anschließend fraktionierend destilliert.

Ausbeute an 2-Brom-4-methylthiazol: 365 g (89 %), farblose Flüssigkeit Sdp.: 51 °C (0,1 mbar)
¹H-NMR (400 MHz, DMSO-d₆): δ = 2,49 (s, 3H, CH₃), 6,85 (s, 1H, Thiazol).
¹³C-NMR (400 MHz, DMSO-d₆): δ =17,25, 117,21, 134,90, 153,46.
MS (CI): (m/z) = M⁺ 179, C₄H₄BrNS: 178,05.

### Beispiel II-B

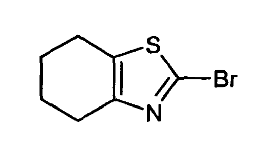

In eine Lösung von 12,02 g (77,41 mmol) 2-Thiocyano-cyclohexanon (siehe z. B. Ali et al. 1981, J. Chem. Res. Miniprint 8, 2901-2927; Tanabe et al. 1994, Chem. Lett. 12, 2275-2278) in 300 mL Dichlormethan wird bei 0°C für 20 min. HBr eingeleitet und anschließend innerhalb von 10 min. auf Raumtemperatur erwärmt und dabei kontinuierlich HBr eingeleitet. Nach 30 min. wird die Gaseinleitung beendet. Nachdem über Nacht bei Raumtemperatur nachgerührt wurde, wird auf 0°C gekühlt und mit einer verdünnten wässrigen Natriumhydrogencarbonatlösung neutralisiert. Die organische Phase wird abgetrennt, über MgSO₄ getrocknet und das Lösungsmittel am Rotationsverdampfer abdestilliert.

Ausbeute an 2-Brom-4, 5, 6, 7-tetrahydro-benzothiazol: 12,0 g (65,4 %), gelbes Öl.
MS (GC/CI): m/z = 218 (M⁺)
¹H-NMR (400 MHz, CDCl₃): δ = 1.85 (m, 4H), 2,75 (m, 2H), 2,85 (m, 2H).
¹³C-NMR (400 MHz, CH₃CN-d₃): δ = 22,75, 23,09, 23,40, 26,64, 132,34, 132,90, 150,94.

### Anwendungsbeispiele

### Reaktivitätsscreening

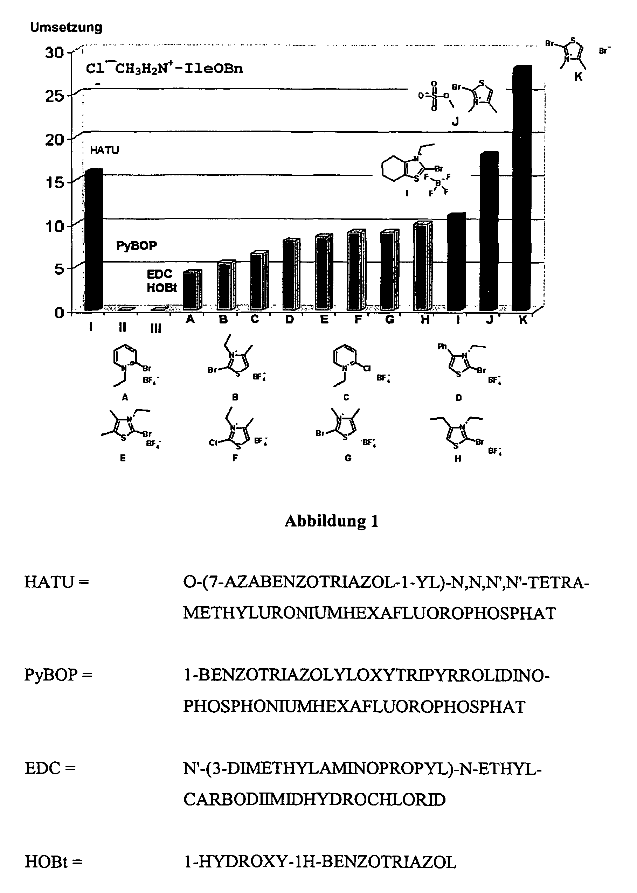

Die relative Reaktivität der neuen Peptidkupplungsreagenzien wurde in einem Reaktivitätsscreening ermittelt. Dazu wurde in 10 ml wasserfreiem Dichlormethan 1.0 eq (54 mg, 0.2 mmol) ClH₂N-MeIle-Bn vorgelegt und mit 1.0 eq (49 mg, 0.2 mmol) Boc-MeIle-OH versetzt. Zu dieser Lösung wurden anschließend bei Raumtemperatur 1.5 eq (0.3 mmol) des zu testenden Kupplungsreagenzes (I, J und K, siehe Abbildung 1) gegeben und daraufhin mit 6 eq (1.2 mmol) Diisopropylethylamin versetzt. Die Reaktionsmischung wurde 3 h bei RT gerührt und zur Reaktionskontrolle im Mikromaßstab aufgearbeitet. Dazu wurden 200 Mikroliter abgenommen und mit 200 Mikroliter einer gesättigten NaHCO₃ - Lösung versetzt. Die organische Phase wurde abgetrennt und mit 1 ml Acetonitril verdünnt. Zur Bestimmung des Edukt/Produkt Verhältnisse wurde 5 Mikroliter dieser Lösung durch HPLC vermessen. Als mobile Phase diente dabei das nachfolgend aufgeführte System: Waters Alliance 2690-System, UV-Detektion, 214 nm; Säule: Waters Xterra C₈, 150x3,9mm, Fluss: 1,2 mL/min; 0-13 min: 95% H₂O, 5% CH₃CN => 5% H₂O, 95% CH₃CN (0,1% TFA in Lösungsmittel). Um einen Vergleich mit bekannten Kupplungsreagenzien zu machen, wurden auch bekannte bzw. nicht erfindungsgemäße Kupplungsreagenzien (I, II, III und B, bzw. A und C-H, siehe Abb. 1) mit dem beschriebenen Verfahren getestet. Der Grad der Umsetzung wird in relativen Einheiten gemessen.

Die durchgeführte Reaktion kann wie folgt beschrieben werden:

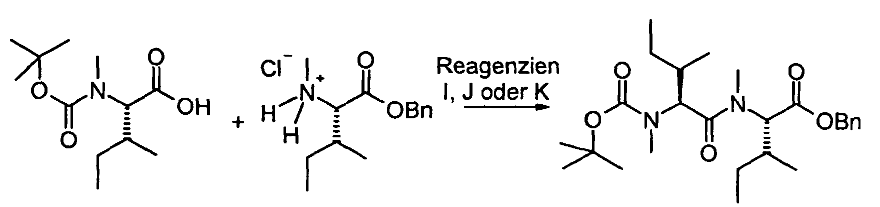

Im Vergleich zu den bekannten Kopplungsreagenzien I, II und III und im Vergleich zu dem bekannten Kopplungsreagenz B zeigen die Kopplungsreagenzien I, J oder K eine sehr gute Reaktivität.

## Patentansprüche

1. Verbindungen der Formel (I) 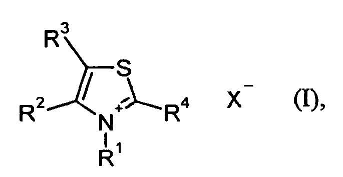
wobei die Reste R¹, R², R³ und R⁴ sowie das Anion X⁻ nachstehende Bedeutungen besitzen:
| **Verbindung** | **R**^{**1**} | **R**^{**2**} | **R**^{**3**} | **R**^{**4**} | **X**^{**-**} |
|---|---|---|---|---|---|
| K | Methyl | Methyl | H | Br | Br |
| J | Methyl | Methyl | H | Br | CH₃OSO₃⁻ |
| I | Ethyl | -(CH₂)₄- | | Br | BF₄⁻ |

2. Verfahren zur Herstellung von Verbindungen gemäß Anspruch 1, **dadurch gekennzeichnet, dass** man in Gegenwart eines Verdünnungsmittels Verbindungen der Formel (II) 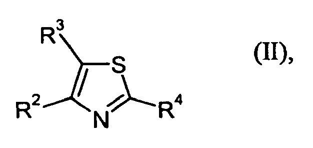
entweder mit Alkylierungsreagenzien der Formel (III)
R¹-X' (III),
oder mit Alkylierungsreagenzien der Formel (IV) umsetzt
(R¹)₃-O⁺ X"⁻ (IV),
wobei in den Formeln (II), (III) und (IV)
R¹, R², R³ und R⁴ die in Anspruch 1 angegebene Bedeutung haben,
X' für Chlor, Brom oder Methylsulfonyloxy und
X'' für Tetrafluoroborat steht
oder
sie mit Wasserstoffperoxid (H₂O₂), Persäuren oder NaOCl oxidiert
und anschließend das Anion X'- gegebenenfalls mit einem Tetrafluoroborwasserstoffsäure beladenen Anionenaustauscher gegen Tetrafluoroborat austauscht.

3. Verwendung von Verbindungen gemäß Anspruch 1 als Kondensationsreagenzien.

4. Verwendung von Verbindungen gemäß Anspruch 1 in der Peptidsynthese.

5. Verbindung der Formel (II-1) 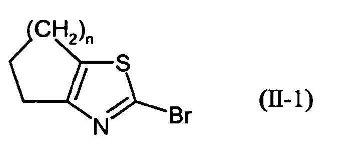
in welcher
n für 2 steht.

6. Verwendung der Verbindung gemäß Anspruch 5 zur Herstellung des Kondensationsreagenzes I gemäß Anspruch 1.

## Revendications

1. Composés de formule (I) 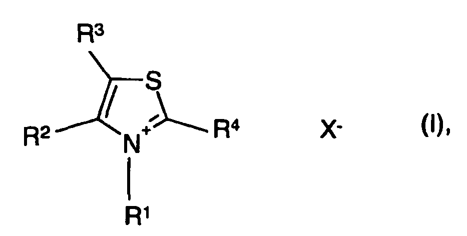
dans laquelle la signification des symboles R¹, R² R³ et R⁴ et la nature de l'anion X⁻ sont les suivantes :
| Composé | R¹ | R² | R³ | R⁴ | X- |
|---|---|---|---|---|---|
| K | méthyle | méthyle | H | Br | Br |
| J | méthyle | méthyle | H | Br | CH₃OSO₃⁻ |
| I | éthyle | -(CH₂)₄- | | Br | BF₄⁻ |

2. Procédé pour la préparation des composés selon la revendication 1, **caractérisé en ce que** l'on fait réagir, en présence d'un diluant, des composés de formule (II) 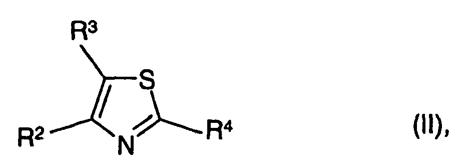
soit avec des réactifs alkylants de formule (III)
R¹-X' (III),
soit avec des réactifs alkylants de formule (IV)
(R¹)₃-O⁺ X"⁻ (IV),
les symboles des formules (II), (III) et (IV) ayant les significations suivantes :
R¹, R², R³ et R⁴ les significations indiquées dans la revendication 1,
X" le chlore, le brome ou un groupe méthylsulfonyloxy et
X" le groupe tétrafluoroborate,
ou bien,
on les oxyde à l'aide du peroxyde d'hydrogène (H₂O₂), de peracides ou de NaOCl,
après quoi on échange le cas échéant l'anion X'⁻ contre l'anion tétrafluoroborate à l'aide d'un échangeur d'anions chargé d'acide tétrafluoroborhydrique.

3. Utilisation des composés selon la revendication 1 en tant que réactifs de condensation.

4. Utilisation des composés selon la revendication 1 dans la synthèse de peptides.

5. Composé de formule (II-1) 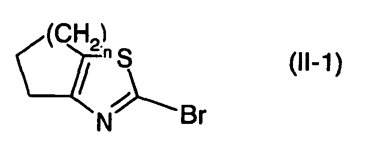
dans laquelle
n est égal à 2.

6. Utilisation du composé selon la revendication 5 pour la préparation du réactif de condensation I selon la revendication 1.

## Claims

1. Compounds of the formula (1) 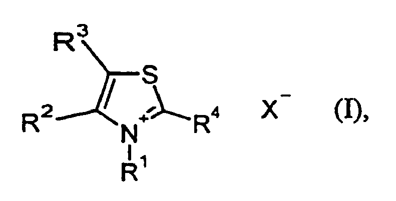
where the radicals R¹, R², R³ and R⁴ and the anion X⁻ possess definitions given below:
| **Compound** | **R**^{**1**} | **R**^{**2**} | **R**^{**3**} | **R**^{**4**} | **X**^{**-**} |
|---|---|---|---|---|---|
| K | methyl | methyl | H | Br | Br |
| J | methyl | methyl | H | Br | CH₃OSO₃⁻ |
| I | ethyl | -(CH₂)₄- | | Br | BF₄⁻ |

2. Process for preparing compounds according to Claim 1, **characterized in that**, in the presence of a diluent, compounds of the formula (II) 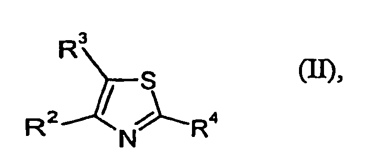
are reacted either with alkylating reagents of the formula (III)
R¹-X' (III)
or with alkylating reagents of the formula (IV),
(R¹)₃-O⁺ X"⁻ (IV)
where in the formulae (II), (III) and (IV),
R¹, R², R³ and R⁴ have the definition specified in Claim 1,
X' is chlorine, bromine or methylsulphonyloxy and
X" is tetrafluoroborate
or
they are oxidized with hydrogen peroxide (H₂O₂), peracids or NaOCl
and subsequently the anion X'⁻ is replaced by tetrafluoroborate, optionally with an anion exchanger loaded with tetrafluoroboric acid.

3. Use of compounds according to Claim 1 as condensation reagents.

4. Use of compounds according to Claim 1 in peptide synthesis.

5. Compound of the formula (II-1) 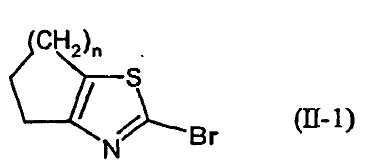
in which
n is 2.

6. Use of the compound according to Claim 5 for preparing the condensation reagent I according to Claim 1.
